**Europäisches Patentamt**

**European Patent Office**

**Office européen des brevets**

(11) Veröffentlichungsnummer: **0 488 140 A1**

## EUROPÄISCHE PATENTANMELDUNG

(21) Anmeldenummer: **91120103.6**

(22) Anmeldetag: **26.11.91**

(51) Int. Cl.⁵: **A61F 7/00**, F17C 9/02

(30) Priorität: **30.11.90 DE 4038131**

(43) Veröffentlichungstag der Anmeldung:
**03.06.92 Patentblatt 92/23**

(84) Benannte Vertragsstaaten:
**AT CH DE IT LI NL**

(71) Anmelder: **MESSER GRIESHEIM GMBH**
**Hanauer Landstrasse 330**
**W-6000 Frankfurt/Main 1(DE)**

(72) Erfinder: **Donnerhack, Andreas, Dr.**
**Crousstrasse 28**
**W-4150 Krefeld(DE)**
Erfinder: **Flohr, Wolfgang**
Uferstrasse 5
**W-6452 Hainburg(DE)**
Erfinder: **Lucht, Heinz Olaf**
**Hülsdonkstrasse 58**
**W-4156 Willich(DE)**
Erfinder: **Pfeil-Schneider, Kurt**
**Johannesfeld 29**
**W-4054 Nettetal(DE)**
Erfinder: **Sassmann, Gerhard**
**Am Schlehdorn 8**
**W-5900 Siegen(DE)**
Erfinder: **Thoma, Klemens**
**Am Kleckers 22**
**W-4150 Krefeld-Hüls 29(DE)**

(54) **Vorrichtung zur Erzeugung eines kalten Behandlungsgases aus flüssigem Stickstoff für die Kryotherapie.**

(57) Vorrichtungen zur Erzeugung eines kalten Behandlungsgases aus flüssigem Stickstoff für die Kryotherapie bestehen aus einem doppelwandigen isolierten Behälter und einer im Behälter angeordneten elektrischen Heizung zur Verdampfung des flüssigen Stickstoffs.

Um die Gefahr einer Beschädigung der Heizung beim Befüllen des Behälters zu beseitigen und eine Reparaturmöglichkeit für die Heizung zu schaffen, ist die Heizung (4) mittels eines am Boden des Innenbehälters (1) angebrachten Befestigungselementes leicht auswechselbar befestigt.

Fig. 1

Die Erfindung betrifft eine Vorrichtung zur Erzeugung eines kalten Behandlungsgases aus flüssigen Stickstoff für die Kryotherapie, mit einem aus Innen- und Außenbehälter bestehenden doppelwandigen isolierten Behälter, nach dem Oberbegriff des Anspruches 1.

Vorrichtungen zur Durchführung der Kryotherapie mittels eines kalten Behandlungsgases, welches aus flüssigen Stickstoff gewonnen wird, sind bekannt. Neben großen Geräten, die für den Einsatz in Kliniken geeignet sind, gibt es auch kleine Geräte mit Speicherbehältern für den flüssigen Stickstoff, die nur einen Rauminhalt von einigen Dutzend Litern besitzen. Diese Geräte sind für kleinere Arztpraxen für nur gelegentlichen Einsatz gedacht. Sofern als Speicherbehälter für den flüssigen Stickstoff Standardbehälter mit enger Halsöffnung verwendet werden, bereitet die elektrische Heizung gelegentlich Schwierigkeiten, da die Heizung im Bodenbereich des Innenbehälters installiert werden muß. Wird sie mittels einer Tauchleitung an einem auf den Behälterhals aufzusetzenden Gehäuse befestigt, wie es das deutsche Gebrauchsmuster 84 27 439 zeigt, muß die Heizung bei jeder Befüllung mit flüssigem Stickstoff aus den Behälter genommen werden. Hierbei kann sie leicht beschädigt werden. Das deutsche Patent 36 21 425 zeigt eine derartige Vorrichtung, bei der die Heizung ein auf dem Behälterinnern befestigtes selbstregulierendes Heizband ist. Hierbei besteht zwar keine Gefahr der Beschädigung der Heizung bei Befüllen, im Falle einer nötigen Reparatur ist sie jedoch nur schwer oder überhaupt nicht zugänglich.

Der Erfindung liegt daher die Aufgabe zugrunde, bei einer aus einem doppelwandigen isolierten Behälter und einer elektrischen Heizung bestehenden Vorrichtung zur Erzeugung eines kalten Behandlungsgases aus flüssigem Stickstoff die Heizung so zu gestalten, daß sie beim Befüllen nicht beschädigt werden kann und zu Reparaturzwecken leicht aus dem Behälter entnommen werden kann.

Ausgehend von dem im Oberbegriff des Anspruches 1 berücksichtigen Stand der Technik ist diese Aufgabe erfindungsgemäß gelöst mit den im kennzeichnenden Teil des Anspruches 1 angegebenen Merkmalen.

Vorteilhafte Weiterbildungen der Erfindung sind in den Unteransprüchen angegeben. Erfindungsgemäß wird demnach die Heizung leicht lösbar mittels eines Befestigungselementes auf dem Boden des Innenbehälters angebracht. Als Befestigungselement wird ein mit einem Innengewinde versehenes Gewindestück bevorzugt, welches auf den Boden des Innenbehälters geschweißt wird. Ein solches Gewindestück ist bei vielen Behältern bereits vorhanden, da es während der Montage als Hilfsmittel zum Aufbringen der Isolation dient. Die Erfindung bietet in solchen Fällen den Vorteil, daß auch in älteren Behältern nachträglich eine Heizung in der erfindungsgemäßen Weise befestigt werden kann. Es sind jedoch auch beliebige andere Verbindungen möglich, beispielsweise Steckverbindungen in Form von Bajonettverschlüssen oder magnetische Halterungen. Das Einsetzen und Herausnehmen der Heizung erfolgt mittels eines speziellen Werkzeuges durch den Behälterhals. Es ist unmittelbar ersichtlich, daß die Erfindung vor allem für kleine Normbehälter mit Enghalsausführung und wenigen Dutzend Litern Rauminhalt von Vorteil ist.

Die Heizung wird zweckmäßigerweise als Heizpatrone mit Wärmetauscherrippen ausgebildet. Um Überhitzungen bei völliger Entnahme des flüssigen Stickstoffs zu vermeiden, ist es vorteilhaft, an der Heizpatrone einen Thermofühler mit Sicherheitsabschaltung zur Temperaturbegrenzung anzubringen. Das Kabel zur Zufuhr der elektrischen Energie wird durch den Behälterhals nach außen geführt. Hierzu ist es vorteilhaft, wenn in den Behälterhals ein eigenes Befüllrohr eingesetzt wird, an dessen Außenseite das Kabel verläuft. Eine Beschädigung des Kabels beim Befüllen ist dadurch ausgeschlossen.

Die Zeichnungen veranschaulichen ein Ausführungsbeispiel der Erfindung.

Es zeigen:

Fig.1    einen Schnitt durch die gesamte Vorrichtung,

Fig.2    eine Heizpatrone mit Wärmetauscher,

Fig.3    einen Teil des Bodens des Innenbehälters mit einem als Gewindestück ausgebildeten Befestigungselement,

Fig.4    einen Schnitt durch die Halsöffnung in vergrößertem Maßstab.

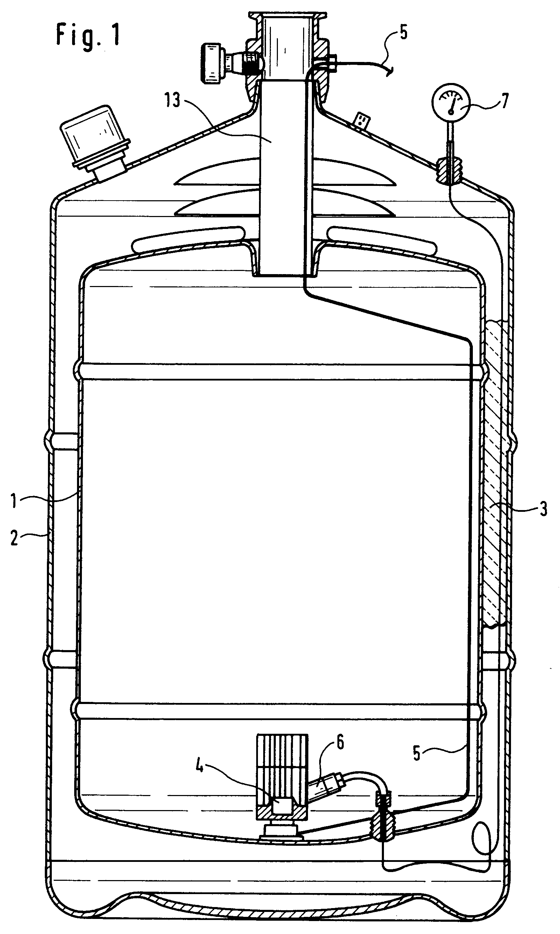

Die in Fig.1 im Schnitt dargestellte Vorrichtung besteht aus einem Innenbehälter 1, einem Außenbehälter 2 und dazwischen angebrachter Isolation 3. Es handelt sich hierbei um einen Standardbehälter in Enghalsausführung. Auf dem Boden des Innenbehälters 1 ist eine Heizpatrone 4 gemäß der Erfindung lösbar befestigt. Die Zufuhr der elektrischen Heizenergie erfolgt durch ein abgeschirmtes Kabel 5, welches durch den Behälterhals nach außen geführt ist. Am Boden des Innenbehälters 1 ist ferner eine nach dem hydropneumatischen Meßprinzip arbeitende Füllstandsanzeige 6 angeordnet. Die Zuleitung ist durch die Isolation 3 geführt und endet im Anzeigegerät 7.

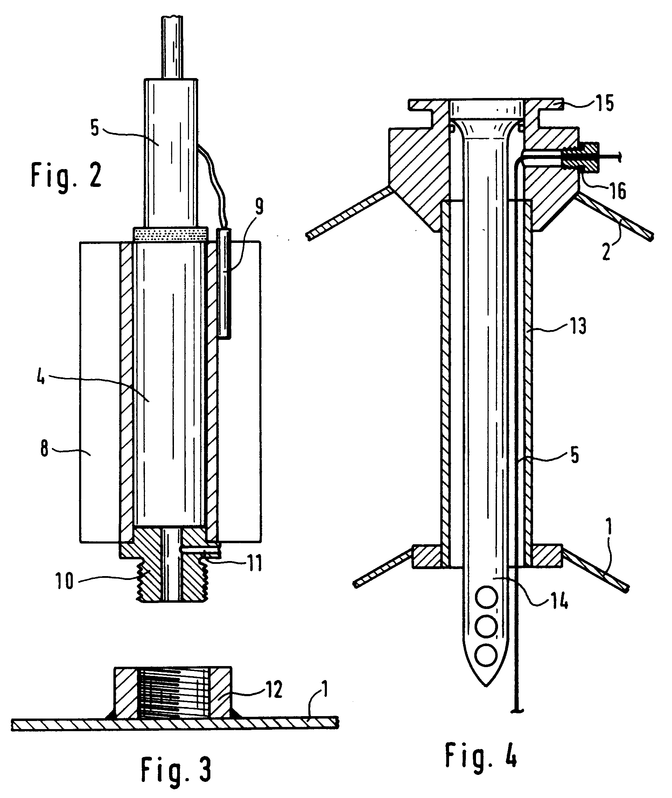

In den Fig. 2 und 3 ist die Heizpatrone 4 vergrößert dargestellt. Die Heizpatrone 4 ist von einem Wärmetauscher 8 in Rippenform umgeben. An der Heizpatrone 3 ist ein Temperaturfühler 9 angebracht, welcher zu einer nicht dargestellten Sicherheitsabschaltung führt. Diese bewirkt, daß nach dem völligen Verdampfen des flüssigen Stickstoffs die Heizung abgeschaltet wird. Am unteren Ende der Heizpatrone befindet sich ein Gewindestück 10 mit Außengewinde, welches eine Druckentlastungsbohrung 11 aufweist. In Fig.3 ist das zugehörige Gewindestück 12 mit Innengewinde dargestellt, welches auf den Boden des Innenbehälters 1 geschweißt ist und in welches die Heizpatrone 4 mit dem Gewindestück 10 eingeschraubt wird.

Fig.4 zeigt im vergrößerten Maßstab das Halsrohr 13, welches den Innenbehälter 1 mit dem Außenbehälter 2 verbindet. In das Halsrohr 13 ist ein Befüllrohr 14 eingesetzt, welches mit dem Halsrohr 13 einen Ringspalt bildet. Durch diesen Ringspalt ist das abgeschirmte Kabel 5 durch den Behälteranschluß 15 und eine Dichtung 16 nach außen geführt. Der Behälteranschluß 15 dient zum Befestigen eines nicht näher dargestellten Gehäuses mit weiteren Bedienelementen und eines Behandlungsschlauches.

Die Heizpatrone 4 kann leicht mittels eines durch das Halsrohr 13 geführten speziellen Werkzeuges in das Gewindestück 12 eingeschraubt werden. Ebenso einfach kann sie, beispielsweise zu Reparaturzwecken, wieder abgeschraubt und durch das Halsrohr 13 entnommen werden. Eine Beschädigung des Behälters ist hierbei ausgeschlossen. Das Halsrohr 13, durch welches der Behälter befüllt wird, bleibt frei zugänglich. Eine Beschädigung der Heizung durch den Befüllvorgang ist ebenfalls ausgeschlossen, insbesondere dann, wenn ein Befüllrohr 14 vorgesehen wird. Das Befüllrohr 14 ist Jedoch nicht unbedingt erforderlich. Es kann weggelassen werden, wenn der volle Querschnitt des Behälterhalses zur Entnahme des tiefkalten Behandlungsgases frei bleiben soll.

## Patentansprüche

1. Vorrichtung zur Erzeugung eines kalten Behandlungsgases aus flüssigem Stickstoff für die Kryotherapie, mit einem aus Innen- und Außenbehälter bestehenden doppelwandigen isolierten Behälter für die Speicherung des flüssigen Stickstoffes und einer im Behälterinnern angeordneten elektrischen Heizung, dadurch gekennzeichnet,
daß die Heizung mittels eines am Boden des Innenbehälters (1) angebrachten Befestigungselementes leicht auswechselbar befestigt ist.

2. Vorrichtung nach Anspruch 1,
dadurch gekennzeichnet,
daß das Befestigungselement ein mit einem Innengewinde versehenes Gewindestück (12) ist.

3. Vorrichtung nach Anspruch 1,
dadurch gekennzeichnet,
daß das Befestigungselement als Steckverbindung ausgebildet ist.

4. Vorrichtung nach Anspruch 1,
dadurch gekennzeichnet,
daß das Befestigungselement als Magnetverbindung ausgebildet ist.

5. Vorrichtung nach einem der Ansprüche 1 bis 4,
dadurch gekennzeichnet,
daß die Heizung als Heizpatrone (4) mit Wärmetauscher (8) ausgebildet ist.

6. Vorrichtung nach Anspruch 5,
dadurch gekennzeichnet,
daß an der Heizpatrone ein Thermofühler (9) zur Temperaturbegrenzung angeordnet ist.

**Fig. 1**

Fig. 2

Fig. 3

Fig. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | DE-U-9 002 065 (WESTFALEN AG)<br>* page 5, line 24 - line 32 *<br>* page 6, line 19 - line 29 *<br>--- | 1-6 | A61F7/00<br>F17C9/02 |
| Y | GB-A-1 148 062 (BRITISH AIRCRAFT CORP. LTD.)<br>* page 1, line 61 - line 65; claim 1; figure 1 *<br>--- | 1-6 | |
| A,D | EP-A-0 250 863 (MESSER GRIESHEIM GMBH)<br>* column 4, line 38 - line 45; figure 1 *<br>--- | 1,3 | |
| A | US-A-4 313 306 (TORRE)<br>* column 3, line 17 - line 26 *<br><br>----- | 1 | |
| | | | TECHNICAL FIELDS SEARCHED (Int. Cl.5)<br><br>A61F<br>F17C<br>A61B |

The present search report has been drawn up for all claims

| Place of search | Date of completion of the search | Examiner |
|---|---|---|
| THE HAGUE | 21 FEBRUARY 1992 | GLAS J. |

EPO FORM 1503 03.82 (P0401)